# EUROPEAN PATENT SPECIFICATION

(11) **EP 0 531 096 B1**
(45) Date of publication and mention of the grant of the patent: **29.10.1997**
(21) Application number: 92307938.8
(22) Date of filing: 01.09.1992
(51) Int. Cl.: A61F 13/00, A61F 13/15

(54) **Composite fabrics**
Textilverbundstoffe
Tissus composites

(30) Priority: 02.09.1991 GB 9118737
(43) Date of publication of application: 10.03.1993
(73) Proprietor: McNEIL-PPC, INC., Milltown New Jersey 08850 (US)
(72) Inventor: Elves, John, 5801 KB Venray (NL)
(74) Representative: Fisher, Adrian John

(56) References cited:
- EP-A- 0 151 018
- DE-A- 1 492 362
- GB-A- 2 078 527
- GB-A- 2 099 704
- US-A- 2 862 251
- US-A- 3 122 142
- US-A- 4 808 467

## Description

The present invention relates to absorbent composite fabrics, especially for use as or in non-adherent wound dressings.

Several characteristics of wound dressings are believed to assist wound healing. For example, the dressing should be sufficiently strong and dense to protect the wound from contamination. The dressing should be air-permeable to allow the wound to breathe. The dressing should be highly absorbent and have a high rate of fluid uptake in order to remove exudate from the wound surface. The dressing should be non-adherent to enable it to be removed or replaced without interiering with wound healing and without discomfort. The dressing should have minimal tendency to shed lint, fibres or fragments into the wound, since such particles can interfere with wound healing. The wound dressing should preferably maintain a humid atmosphere at the wound surface since this assists healing. Finally, a wound dressing should have good fluid retention properties to prevent absorbed fluids leaking from the dressing and staining bedclothes, etc.

It will readily be appreciated that absorbent fabrics having the properties listed above will be useful as or in many other products besides non-adherent wound dressings, such as sweat-absorbent pads or linings, baby garments, medical drapes, feminine hygiene products and incontinence pads.

One approach to the design of non-adherent wound dressings is the use as or in wound dressings of absorbent composite fabrics comprising a layer of fluid-absorbent hydrophilic material bonded to a layer of hydrophobic material. As will be appreciated by those skilled in the art, the term "hydrophilic" is used to describe materials which are wetted by water (i.e. the surfaces of the materials have contact angles with water less than 90°), while the term "hydrophobic" is used to describe materials which are not wetted by water (i.e. the surfaces of hydrophobic materials have contact angles with water greater than 90°).

Published specification EP-A-0151018 (Johnson & Johnson) discloses absorbent composite fabrics comprising a hydrophobic fleece of fibrous material in the weight range 100 to 500g/m² surrounding and enclosing a layer of fluid-absorbent material such as peat moss board in the weight range 100 to 1000g/m². The two layers are joined by passing barbed needles into and out of the adjacent layers in a reciprocating motion, a process known in the art as needling. The effect of needling is to cause local penetration at the needling points of the fibres of the hydrophobic fleece into the fluid-absorbent layer, thereby joining the layers and forming wicks of hydrophilic material; fluids from the wound are absorbed through the hydrophobic fleece into the hydrophilic layer *via* the wicks.

This document forms the preamble of independent claims 1 and 18.

In order to maximise the strength of the above composite fabrics, and to maximise the rate of fluid transport through the hydrophobic fleece, it is desirable to have a high needling density per unit area of the composite fabric. However, a high needling density aggravates the problem of fluid from the absorbent material seeping back through the wicks and partially wetting the surface of the hydrophobic fleece. This problem is known in the art as wet-back. A satisfactory needling density may be combined with a low level of wet-back by use of a thick hydrophobic layer. A thickness of at least 80g/m² and preferably 100-500g/m² is used in practice. The thick hydrophobic fleece does not increase the total water absorbency of the fabric but merely increases the cost and bulk of the fabric and makes it less flexible.

GB-A-1130857 describes a wound dressing material comprising a contact layer forming one surface of the material and formed from fibres of a thermoplastic resin which is hydrophobic, and an absorptive layer formed from material which is absorptive, at least some of the fibres from the contact layer extending into the material of the absorptive layer. The layers are joined by needling.

GB-A-2078527 describes an absorbent article, such as a disposable diaper. The article has a laminated structure including a porous top sheet having a basis weight of 15-20g/m², a conventional fibrous absorbent layer, and a layer of superabsorbent polymer behind the fibrous layer. The amount of wet back can be reduced by using hydrophobic fibres in the porous top sheet.

US-A-2862251 describes the preparation of porous non-woven fabrics by treating a non-woven fabric with a large number of small diameter water jets.

The present invention provides an absorbent composite fabric formed from a water-permeable hydrophobic layer of fibrous material and a water-absorbent fibrous second layer, the first and second layers being bonded together by entanglement of fibres of the first layer in fibres of the second layer, and characterized in that the first layer has a weight of from 15 to 40 g/m².

The entanglement is typically hydraulic entanglement, achieved by directing high-pressure jets of water at the surface of the first layer while the fabric is supported on a mesh backing. Hydraulic entanglement provides a strongly bonded composite without needling, and has the added advantage that the hairiness of the hydrophobic surface of the composite fabric is diminished.

Absorbent composites bonded by entanglement rather than needling have the advantage that with suitably chosen fibrous materials, if the weight of the water-permeable first layer is between 15 and 40g/m², the layer not only prevents wet-back but also retains sufficient permeability to allow water-based fluids to pass rapidly through the layer and be absorbed by the water-absorbent layer. As a result, the composite fabric having a weight of the water-permeable first layer of 15-40g/m² will have excellent properties for use as or in wound dressings or as a facing for a wound dressing assuming that the fabric is applied with the hydrophobic layer contacting the wound. The properties will include light weight, flexibility, strong bonding between the composite layers, high specific absorbency, rapid uptake of fluid exudates from the wound through the hydrophobic layer into the water-absorbent layer, and no wet-back. Avoidance of wet-back largely prevents adherence of the composite fabric to the wound. Furthermore, both layers of the composite fabric generally contain only staple fibres, which are much less prone to shed into the wound than the shorter pulp fibres widely used in wound dressings according to the prior art. The hydrophobic layer faces the water jets during the hydraulic entangling process with the result that the fibre ends at the surface of the hydrophobic layer are pushed into the fibre structure by the water pressure, resulting in minimal visible hairiness of the hydrophobic surface in the finished fabric. The minimal hairiness of the hydrophobic surface that results from hydraulic entanglement of the composite fabric further reduces the adherence of the fabric to the wound.

The absorbent composite fabrics according to the present invention are especially suitable for use as or in medical blankets, sheets, sweat-absorbent linings for wetsuits or shoes, medical drapes, absorbent feminine hygiene products such as intermenstrual pads, in incontinence protection products, and any other applications where absorbent fabrics or facings that maintain a dry and nonadherent surface are needed.

The fibres of the first layer are usually staple fibres and will generally be in the length range 15-60mm and preferably 35-40mm. The fibres should be sufficiently thick to provide a water-permeable first layer, which generally implies fibres having a thickness of at least about 0.5 decitex and preferably 1 decitex or more. Typically the fibres are in the thickness range 1-4 decitex:, and preferably 1.5-2.5 decitex.

The fibres of the first layer may be inherently hydrophobic, in that they are formed from a hydrophobic polymer such as polypropylene. Alternatively, the fibres may be hydrophobic by virtue of a hydrophobic finish applied to the surface of the fibres, either before or after entanglement of the composite. For example, the fibres of the first layer may be formed from a polyester such as polyethylene terephthalate which is normally manufactured with a hydrophilic coating to facilitate handling and carding. Such fibres are then further coated with a hydrophobic finish before or after entanglement of the composite fabric. It will be apparent to the skilled reader that many other fibres, with or without a hydrophobic finish, could be used in the hydrophobic layers. The optimum weight of the water-permeable first layers needed to combine water permeability with minimal wet-back may vary slightly according to the fibres being used, but will generally be in the range 15-40g/m², and for the polypropylene and polyester fibres described above will typically be in the range 20-30g/m².

The composition of the water-absorbent fibrous second layer can vary widely while remaining within the scope of the present invention. Preferably, the water-absorbent fibrous second layer comprises mainly hydrophilic staple fibres of viscose, modified viscose such as trilobal cross-section viscose, cotton or solvent spun cellulose, or mixtures of these. The second layer may also contain up to 30% by weight of fibres that do not absorb water, such as hollow-section polyester fibres. The non-absorbent fibres bulk the layer, improve its flexibility, and improve its porosity when wet. The weight of the second layer is usually between 20 and 120g/m². The exact weight range chosen for the fibrous second layer will depend on the application that is envisaged for the composite fabric. The second layer will typically be in the weight range 20-30 g/m² in cases where the composite fabric is used as a dry and nonadherent facing for absorbent products such as feminine hygiene pads or absorbent wound dressings. In this case the second layer acts primarily as a fluid transfer layer to bridge the transfer of fluid into the absorbent product. In other applications, where substantial liquid absorbency of the second layer is required such as in sweat-absorbent bands and linings, baby wraps and the like, the weight of the fibrous second layer will typically be 90-120 g/m². Another example is the three-layer sandwich structured composite described below, which is especially suitable for use as a wound dressing, and in which the absorbent fibrous second layer has a weight typically in the range 60-120 g/m².

The lengths and thicknesses of the staple fibres in the water-absorbent fibrous second layer are not critical. The fibre length of the hdrophilic fibres is preferably in the range 15 to 60mm and preferably 30 to 50mm. The weight of the hydrophilic fibres is normally in the range 1 to 6 decitex and preferably 1.3 to 3 decitex.

The water-absorbent second layer may also contain oligodynamic silver salts such as silver chloride and silver sulphadiazine or other antiseptics. The antiseptic may conveniently be in the form of antiseptic fibres, such as viscose fibres containing chitin. The water-absorbent second layer may also contain humectants such as glycerol.

A composite fabric, provided by the present invention, that is especially suitable for use as a wound dressing is formed from two water-permeable first layers of fibrous material each having a weight of from 15 to 40 g/m², the water permeable first layers being hydrophobic and being bonded one on each side of a water-absorbent second layer by entanglement of fibres of the first layers in fibres of the second layer. The compositions and weights of the layers are as described above. The resulting fabrics have a symmetrical sandwich structure with fluid-permeable hydrophobic layers on the outside. Thus, either face of these fabrics may be applied to the surface of a wound when the fabrics are used as wound dressings. Furthermore, the two hydrophobic layers on either side of the absorbent second layer substantially prevent leakage of fluid out of the fabric.

Any of the fibres used in the layers of the composite fabrics may be coloured, and the fabrics may be printed or patterned on one or both surfaces.

The present invention further provides a method of manufacture of absorbent composite fabrics, comprising the steps of: forming a layered web comprising a water-permeable first layer of a first fibrous material and a second fibrous layer containing water absorbent fibrous material; entangling the fibres of the first and second layers of the layered web at the interface between the first and second layers by supporting the web structure on a water permeable mesh with the said second layer closest to the mesh and moving the layered web and mesh relative to a plurality of water jets while the jets are directed at the surface of the said first layer of the layered web; and drying the resulting composite fabric, characterized in that the first layer has a weight of from 15 to 40 g/m².

The first and second layers may comprise a plurality of simple carded webs.

The hydraulic entanglement step may be carried out by placing the layered web on a substantially flat and horizontal mesh conveyor belt, with the second layer of the layered web facing downwards, and conveying the layered web linearly under a plurality of water jets, which may be arranged in a series of rows. This method of hydraulic entanglement achieves substantially continuous entanglement of fibres at the interface between the layers of the composite, resulting in a strongly and uniformly bonded composite fabric. Furthermore, because the water jets are directed onto the surface of the first layer of the layered web, the entanglement is mainly of fibres from the first layer extending into the second layer. The first layer of the resulting composite and in particular the surface of the first layer of the composite, remain substantially free from fibres of the water absorbent fibrous material from the second layer of the composite fabric.

Alternatively, the layered structure may be placed on a mesh belt supported on an open-surface cylinder with the second layer of the structure facing towards the inside of the cylinder. The mesh belt is then rotated relative to water jets located around the circumference of the cylinder, resulting in hydraulic entanglement as described above.

Methods to produce nonwoven fabrics by hydraulic entanglement of staple fibres are well known in the nonwoven fabrics art and are described, for example, in US-A-3485706, the disclosure of which is incorporated herein by reference.

Where the fibres of the first fibrous material are not hydrophobic they may be provided with a hydrophobic finish as part of the process of manufacturing the composite fabric. The hydrophobic finish may, for example, be applied by printing onto the surface of the composite structure either before or after the entangling step. The hydrophobic finish may be a commercial aqueous fluorocarbon finish such as SCOTCHBAN 824 (Trade Mark) and may optionally be thickened with hydroxy ethyl cellulose or other thickeners prior to application in order to prevent percolation of the hydrophobic finish into the water-absorbent second layer.

The two-ply composite fabric manufactured in this way can undergo second and subsequent entangling steps with second and subsequent fibrous webs, which may themselves be layered, in order to produce three-ply or multi-ply composite fabrics.

Absorbent composite fabrics and methods of manufacture according to the present invention will now be described in more detail, by way of example, with reference to the accompanying drawings, in which:
- Figure 1: is a cross section through a two-layer absorbent composite fabric;
- Figure 2: is a detail view of part of Figure 1;
- Figure 3: is a cross-section through a three-layer absorbent composite fabric;
- Figure 4: is a schematic drawing of a method of manufacture according to the present invention for making the fabric of Figure 1; and
- Figure 5: is a schematic drawing of a method of manufacture by a continuous process according to the present invention for making the fabric of Figure 3.

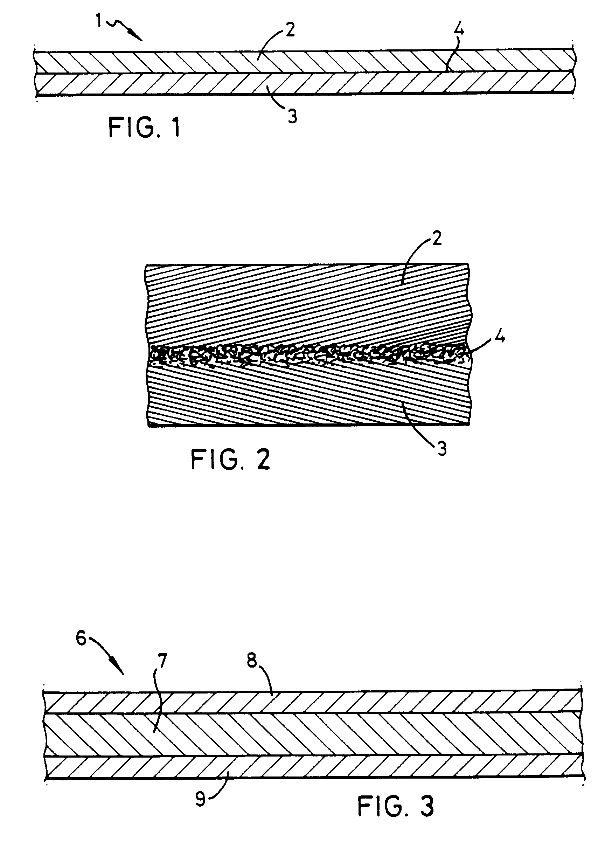

Referring to Figure 1, the composite fabric 1 consists of two nonwoven layers 2, 3 bonded together at an interface 4. Layer 2 is the water-permeable first layer composed of hydrophobic fibres such as polypropylene. The fibre length of the polypropylene is in the range 15-60mm and typically 35-40mm. The fibre thickness is 1-4 decitex and typically 1.5-2.5 decitex. The layer weight of the layer 2 is 15-40g/m² and typically 25g/m². The layer 3 of the composite fabric is the water-absorbent layer and must therefore comprise mainly hydrophilic fibres and preferably water-absorbent fibres. Staple viscose fibres are preferred. The weight of layer 3 ranges from 20 to 200g/m² and is typically 20 to 30g/m² for composite fabrics used as facings and 90 to 120 g/m² for composite fabrics requiring substantial absorbency of the water-absorbent layer.

The low weights of the layers 2, 3 mean that the fabric 1 is light and flexible. The fabric is of course compressible, but in its uncompressed state is generally about 0.5-2.0mm thick. The surfaces of the fabric 1 may be smooth or bear an indented rib or mesh pattern.

Referring to Figure 2, the layers 2 and 3 are joined by continuous entanglement of the fibres of the two layers at the interface 4. Figure 2 is also intended to make clear that the entanglement resulting from continuous hydraulic entanglement as described below is mainly due to hydrophobic fibres extending into the hydrophilic layer 3, and that very few hydrophilic fibres extend into the hydrophobic layer 2. The minimal penetration of fibres from the hydrophilic layer into the hydrophobic layer arises because it is the hydrophobic layer which faces the water jets in the entanglement process. As a result, even very thin hydrophobic layers in the 15-40g/m² weight range retain outer surfaces that are substantially free from hydrophilic fibres. The hydrophobic surface of the composite fabric 1 after entanglement will comprise no more than 5% of hydrophilic fibres and usually no more than 1% of hydrophilic fibres.

The three-layer composite fabric 6 shown in Figure 3 is especially suitable for use as or in wound dressings. The composite fabric 6 comprises a water-absorbent layer 7 sandwiched between two water-permeable hydrophobic layers 8, 9. The compositions and bonding of the layers 7, 8, 9 are similar to those described above for the two-layer laminated fabric 1. The weight of the central water-absorbent layer is typically 60-120 g/m². The water-absorbent layer 7 may itself be a two-ply or multi-ply layer. The sandwich structure need not be symmetrical. The three-layer laminated fabric 6 has hydrophobic layers 8, 9 on both faces, and thus either face of the composite 6 may be applied to a wound in or as a wound dressing. Furthermore, the two hydrophobic layers 8, 9 on either side of the water-absorbent layer 7 trap absorbent fluids in the water-absorbent layer 7 and substantially prevent the absorbed fluids from seeping out and staining clothes or bedclothes.

The above-described composite fabrics 1, 6 having water-permeable hydrophobic layers 2, 8, 9 consisting essentially of polypropylene fibres may be unsuitable for certain applications, in that the composite fabrics 1, 6 cannot be sterilised by gamma radiation because gamma radiation degrades polypropylene. In addition, polypropylene softens above 130°C, so drying and/or heat sterilisation of the above composite fabrics must normally be carried out below 130°C. Accordingly, in an alternative embodiment of the composite fabrics according to the present invention, the above-described polypropylene fibres are replaced by polyester fibres of similar dimensions. The preferred polyester is polyethylene terephthalate (PET), which is considerably more resistant to elevated temperatures and gamma radiation than polypropylene. Since PET fibres are normally manufactured with a hydrophilic surface finish for ease of carding, the composite fabrics according to this embodiment of the present invention are further provided with a hydrophobic fluorocarbon finish applied to the polyester fibres of the composite fabric after the continuous entanglement of the composite fabric. A suitable hydrophobic finish widely known in the textile finishing art is SCOTCHBAN 824 (Trade Mark).

The above-described viscose fibres may be replaced by or mixed with cotton or modified viscose or solvent spun cellulose. Special viscose fibres such as trilobal viscose, which has extremely high water absorbency, may be used. The water-absorbent layers 3, 7 themselves may have a composite structure.

It has been found especially advantageous to add up to approximately 30% by weight of staple fibres that do not absorb water, such as hollow polyester fibres to the water-absorbent layers 3, 7. The hollow polyester fibres bulk the water-absorbent layers and make them more flexible. The hollow polyester fibres maintain the porosity of the water-absorbent layers when the layers are wet, thereby increasing the total water absorbency of the water-absorbent layers. Furthermore, incorporation of thermoplastic fibres such as polyester into the hydrophilic layers 3, 7 enables the edges of the layers to be heat-sealed to prevent any possibility of delamination at the edges or leakage of fluid out of the edges of the composite fabrics 1, 6. The hollow polyester fibres are typically 20-60mm long and as thin as practicable, typically about 4 decitex.

The water-absorbent layers 3, 7 may be impregnated with an antiseptic such as silver chloride or silver sulphadiazine. They may also contain a humectant such as glycerol to retain water in the composite fabrics 1, 6 and thereby maintain a humid atmosphere at the wound surface when the fabrics are used as or in a wound dressing.

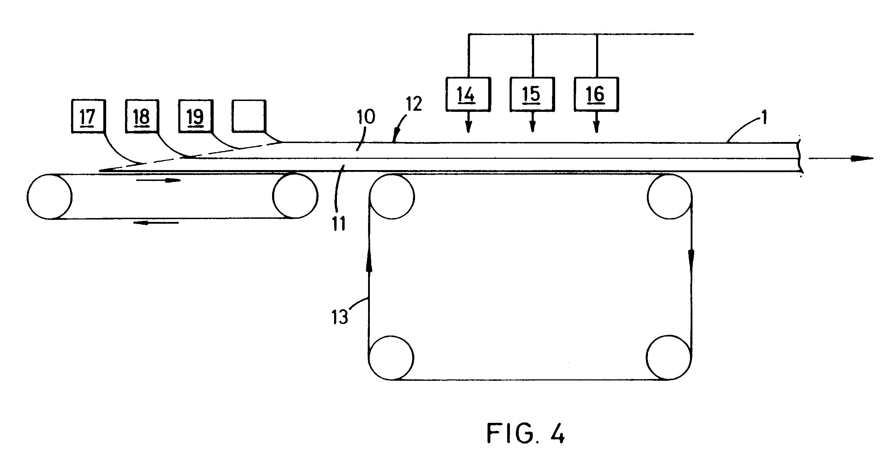

The absorbent composite fabric 1 may be manufactured by the method according to the present invention shown schematically in Figure 4. Referring to the drawing, the first stage is to form a loosely bonded nonwoven layered web 12 comprising a first layer 10, destined to form the water-permeable hydrophobic layer 2 of the absorbent composite fabric 1, and a second layer 11 destined to form the water-absorbent second layer of the fabric 1. The compositions and weights of the first and second layers 10, 11 are as described above for respective layers 1, 2.

The nonwoven web 12 is formed by a series of carding devices 17, 18, 19 mounted in series overall a conveyor belt. Typically there are ten carding devices. Each carding device combs fibres into a fine web weighing up to 12g/m² and deposits this web on the conveyor belt. By stacking fine webs the series of carding devices builds up the nonwoven layered web 12. It will readily be understood how the differing compositions of the layers 10, 11 of the nonwoven layered web 12 are achieved by feeding the carding devices 17, 18, 19 with different fibre compositions.

The nonwoven web 12 is placed flat on a moving mesh belt 13 with the water absorbent second layer 11 of the web in contact with the belt 13. The moving mesh belt 13 transports the nonwoven web 12 past a series of water jets directed onto the surface of the first layer 10. The mesh belt normally travels at a speed relative to the water jets of 2m/min to 60m/min and preferably from 10m/min to 50m/min. The water jets are arranged in rows 14, 15, 16 and are supplied with water at a pressure of about 35 bar (525 psi). The number of rows needed to achieve satisfactory entanglement of the first and second layers increases with the line speed, for example 9 rows are suitable for a line speed of 20m/min and 16 rows are suitable for a line speed of 40m/min. Each row of jets will usually comprise 5-20 jets/cm with a jet orifice size of 50-500µm, preferably 100-200µm.

The mesh of the moving mesh belt 13 is typically woven from strands of plastics or stainless steel. The size characteristics of the mesh used for the moving mesh belt 13 are important in determining the properties of the finished composite fabric. This is because the pressure of the entangling water jets forces the layered web against the mesh during the entangling step and redistributes some of the web fibres, with the result that the finished fabric bears a pattern of indentations, each indentation corresponding to a "knuckle" in the mesh where two of the strands making up the mesh cross. The depth of the indentations depends mainly on the diameter of the strands making up the mesh and the density of the indentations depends on the mesh size of the mesh weave. Large indentations provide a pathway for rapid penetration of liquid through the first hydrophobic layer of the fabric. However, large indentations will also tend to give rise to increased -wet-back. Conversely, a mesh belt woven from fine strands will result in a finished composite fabric that has only small indentations, resulting in longer liquid penetration times and less wet-back. It follows that the choice of belt for the entangling step will depend on the desired characteristics for the finished composite fabric. If the mesh belt 13 is a plain square weave mesh it will generally lie in the mesh size range 10 x 10 mesh to 40 x 40 mesh (4 x 4 strands/cm to 16 x 16 strands/cm) and will typically be 20 x 20 mesh (8 x 8 strands/cm). The square weave meshes are generally woven from plastics strands of diameter 0.4 to 0.8 mm. Typically, strands of diameter 0.8 mm are used for the coarse 10 x 10 mesh (4 x 4 strands/cm) square weave, strands of diameter 0.4 mm are used for the fine 40 x 40 mesh (16 x 16 strands/cm) square weave, and strands of diameter 0.6 mm are used for the intermediate 20 x 20 mesh (8 x 8 strands/cm) square weave.

It should be understood that the mesh belt 13 is not limited to square weave meshes but may comprise ribbed weave meshes, or twill weave meshes, or other meshes. Furthermore, the mesh may comprise strands of more than one diameter.

It should also be understood that the method of manufacture of composite fabrics according to the present invention is not limited to the use of the linear entangling apparatus described above. For example, the water permeable mesh may be mounted on an open surface cylinder. The layered web 12 is then placed on the mesh with the water-absorbent layer 11 facing the inside of the cylinder and the cylinder is rotated relative to entangling jets located around the circumference of the cylinder.

Following entanglement, the resulting composite fabric 1 is dries. The drying temperature is generally as high as practicable and typically 130°C for the polypropylene/viscose composite described here. The entangled composite 1 may also undergo sterilisation with steam or ethylene oxide.

Replacement of polypropylene fibres by polyester fibres in the absorbent composite allows the drying step of the manufacturing method to be carried out at temperatures above 130°C. Furthermore, the composite fabric after drying may be sterilised with gamma radiation.

The polyester fibres are initially provided with a hydrophilic finish for ease of handling and ease of carding. Following entanglement and drying of the composite fabric 1 containing such polyester fibres, a hydrophobic finish is printed onto the surface of the polyester-containing layer of the composite fabric. Typically, the hydrophobic finish is applied in an aqueous fluorocarbon suspension such as SCOTCHBAN 824 (Trade Mark) mixed with hydroxy ethyl cellulose or a similar thickener to increase its viscosity to 450-500 centipoise. The mixture is typically printed onto the surface of a polyester-containing layer of the composite fabric following the drying step. The finish is typically printed in a very fine pattern of lines 400-500µm wide and about 1.0mm apart. The average weight of the applied finish after drying is normally in the range of 0.2 to 2g/m². Typically, the dried finish comprises about 0.4g/m² of fluorocarbon and 0.6g/m² of hydroxy ethyl cellulose. The use of a viscous hydrophobic finish makes it possible to coat only fibres of the first fibrous layer with the hydrophobic finish. Percolation of the hydrophobic finish into the water-absorbent layer of the composite is avoided.

The composite fabric 1 may undergo further hydraulic entangling steps with further carded webs. For example, a composite fabric having a two-ply water-absorbent layer may be made by placing a first composite fabric 1 on the water permeable mesh with the hydrophobic first layer 2 of the fabric facing the mesh, carding a second web of water-absorbent fibres on top of the water-absorbent surface of the first composite fabric and then hydraulically entangling the second web and the first composite fabric as described above to provide a composite fabric according to the present invention having a two-ply absorbent layer. This two-step entangling procedure enables composite fabrics according to the present invention and weighing up to 180 g/m² to be manufactured by hydraulic entanglement. The thick two-ply absorbent layers are especially suitable where high liquid absorbency is required.

Alternatively, the composite fabric shown in Figure 3 may also be made by carrying out a second hydraulic entangling step on the composite fabric 1. In this case the composite fabric 1 is placed on the conveyor belt with the hydrophobic first layer facing the conveyor belt, and a second layered web having a water permeable first layer and a water absorbent second layer is formed on top of the composite fabric by carding, with the water-absorbent layer of the second layered web in contact with the water-absorbent layer of the composite fabric. The first and second layers of the second layered web are bonded to each other, and the second layered web is simultaneously bonded to the underlying composite fabric, by hydraulic entanglement carried out as described above.

Where the structure and composition of the second layered web are the same as the structure and composition of the first layered web 12, a substantially symmetrical sandwich structure as shown in Figure 3 is formed by the second entangling operation followed by drying the composite. In other words, the resulting composite fabric has a water-permeable hydrophobic outer layer bonded to each side of a two-ply water-absorbent layer. However, second and subsequent entanglement operations according to the present invention may be used to generate unsymmetrical multi-ply composite fabrics. The multi-ply composite fabrics may be provided on one or both surfaces with a hydrophobic finish as described above.

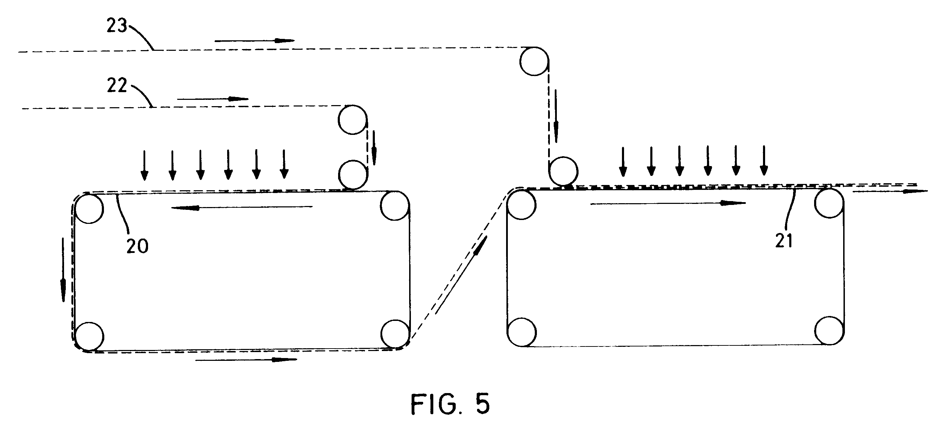

The second and subsequent entanglement steps described above for making multi-ply composite fabrics may be carried out on the composite fabric 1 either immediately following the first entanglement step while the composite fabric 1 is still wet, or after drying the composite fabric 1. Multi-ply composite fabrics can be made in a continuous process by employing a plurality of entangling belts (20, 21) to entangle a plurality of carded webs (22, 23), as shown in Figure 5.

Particular embodiments of the absorbent composite fabrics and method of manufacture according to the present invention are described in and by the following examples:

### Example 1

A composite fabric having an absorbent core and hydrophobic layers on both surfaces, suitable for use as a wound dressing is prepared as follows. The fabric comprises staple viscose fibres of length 38mm and weight 1.7 decitex, hollow polyester fibres of length 60mm and weight 4.0 decitex and standard polyester fibres of length 38mm and weight 1.6 decitex. A combined web with a bottom (second) layer of 45g/m², 75/25wt% viscose/hollow polyester and a top (first) layer of 30g/m² standard polyester is produced by a plurality of carding machines. The web is placed on a moving woven polyester belt with a 20 x 20 mesh (8 x 8 strands/cm) pattern and a strand diameter of 0.6mm with the bottom layer in contact with the belt. Whilst supported on the belt the web is subjected to hydraulic entanglement with water jets at a pressure of 35 bar (525psi) the jets being arranged in 16 rows, each row having 12 jets/cm and the jet orifice size being 178µm (0.007 inches). The speed of the web passing under the jets is 40m/min. After entangling the fabric is dried on steam filled cylinders at 150°C.

This entangled composite fabric is placed back onto the support belt of the entangler with the top (polyester) layer in contact with the belt and an identical combined web is placed on top of it, with the polyester layer on top. The combined web is again hydraulically entangled as above and dried.

Each surface (both 100% polyester) is coated with 40/60 mixture of fluorocarbon (Scotchban FC824) and hydroxyethylcellulose at a level of 1g/m². The coating is applied by gravure printing with a spirally engraved print roller with engraved lines of 0.45mm width, 0.9mm apart. The coating viscosity is 450cps. The resulting fabric has a weight overall of 152g/m².

Composite fabrics prepared in this way have undergone clinical testing as wound dressings for human patients. It has been found that the composite fabrics keep wounds moist, and also are more non-adherent and need to be changed less frequently than conventional wound dressings.

### Example 2

A composite fabric having a thick absorbent layer and a hydrophobic layer on one surface, suitable for use as a baby blanket is prepared as follows. The fabric comprises viscose and standard polyester fibres as in Example 1. A first composite part fabric comprising 30g/m² polyester on one side and 35g/m² viscose on the other side is produced as in Example 1. This fabric is placed back on the support belt of the entangler with the polyester layer in contact with the belt, and a web of 65g/m² viscose is placed on top of it. The support belt for this second stage entangling is 100% polyester with a rib pattern effect (ref. 5710R from Appleton Wire). The entangling conditions are as for Example 1.

To improve its hydrophobicity the polyester surface is coated with a fluorocarbon/hydroxyethylcellulose mixture as in Example 1. The viscose side was given a pink coloured coating for aesthetic and side differentiation purposes.

The fabric thus produced had an at:ractive three dimensional ribbed pattern with a white hydrophobic surface and a pink absorbent surface. The resulting fabric has an overall weight of 132.5g/m².

The absorbent capacity of the fabric thus produced was determined by the standard method of immersing the fabric in water, removing it and allowing it to drip for 30 seconds and reweighing. The fabric was found to have an exceptionally high absorbent capacity of 8g/g, which renders the fabric especially suitable for use in applications where high absorbent capacity per unit weight is required, such as baby blankets.

### Example 3

A light-weight composite fabric suitable for use as a sanitary towel facing is prepared as follows. The fabric comprises viscose fibres of the kind used in Example 1 and polypropylene fibres of length 40mm and weight 2.2 decitex. A combined web with a bottom layer of 40g/m², 75/25 viscose/polypropylene and a top layer of 20g/m² polypropylene with a hydrophobic spin finish (Danaklon soft 71K/L) is produced by a plurality of carding machines. The web is placed on a moving polyester belt with a 16 x 14 mesh (6.3 x 5.5 strands/cm) pattern, and a strand diameter of 0.6mm, with the viscose/polypropylene side in contact with the belt. The web is hydraulically entangled using the conditions described in Example 1, and dried at 130°C. The resulting fabric has an overall weight of 60g/m².

When used as a sanitary towel facing the fabric gives a very soft, compressible, textile feel with zero wet-back. The relatively coarse pattern produced by the support belt during entangling encourages rapid fluid absorption. The polypropylene fibres in the absorbent layer help to promote softness, but their main function is to allow sanitary towel production by heat sealing.

### Example 4

The extent of wet-back and penetration by water of the composite fabric prepared in Example 1 is measured as follows. First, a weighed filter paper (Whatman No. 4 -18.5cm diameter) is placed on a flat surface. A sample (20cm x 20cm) of the composite fabric is placed centrally on top of the filter paper. Then an electric burette is used to dispense 1ml of water onto the centre of the fabric sample. The burette tip is held not more than 5mm above the fabric surface. A stopwatch is started and the time taken for all of the water to be absorbed by the fabric is measured. 30 seconds after all the fluid has been absorbed, a second weighed filter paper is placed on top of the fabric and a padded weight (750 ± 20g,, size 25.5 x 7.5cm) is placed on top of the second weighed filter paper. After a further 30 seconds the weight is removed and the filter papers are reweighed. The weight gain for each filter paper is calculated. The weight gain of the top filter paper is recorded as wet-back; the weight gain of the bottom filter paper is recorded as penetration. The test is repeated with each side of the fabric uppermost.

Tests carried out on the composite fabric of Example 1 gave wet-back and penetration values for either side of less than 0.5g, indicating that only water vapour had permeated back through the hydrophobic surfaces. This was confirmed by the observation that, when the water used for the test was coloured with potassium dichromate, no dichromate was visible on the filter papers after the test.

### Example 5

The extent of wet-back and penetration by water of the composite fabrics prepared in Examples 2 and 3 is measured by a similar method to that described in Example 4 except that wet-back and penetration are determined in two separate steps. Wet-back is measured by placing the fabric on a non-absorbent surface and applying fluid to the hydrophobic side. Only the top filter paper is used. Penetration is measured by laying the fabric on top of a filter paper with the hydrophobic side down, and applying water to the absorbent side.

It was found that the composite fabrics of Examples 2 and 3 exhibit minimal penetration and wet-back, the measured values being only 0.05g.

### Example 6

The liquid absorbency of the composite fabrics prepared in Example 1 is measured as follows. First, a filter paper is placed on a flat, transparent glass plate. The glass plate is positioned with an angled mirror beneath it so that the underside of the filter paper can be observed through the glass plate. Then a 10cm x 10cm sample of the composite fabric which has been sterilized by gamma-irradiation is placed atop the filter paper. On top of the sample of composite fabric there is rested a sheet of Perspex (Registered Trade Mark) of dimensions 125mm x 125mm x 8mm thick and weight 140 ± 10g. A 25mm diameter hole in the Perspex sheet is positioned over the centre of the sample of composite fabric. Water coloured with potassium dichromate is trickled onto the surface of the composite fabric through the hole in the Perspex sheet by means of an electric burette and a funnel almost touching the surface of the fabric. The burette delivers water at the rate of 0.1ml/4 seconds until any part of the filter paper is observed to be wet, indicating that the composite fabric is saturated with water so that excess water is leaking from the fabric. The total volume of water delivered then gives the absorbency of the fabric.

The above measure of absorbency is of practical usefulness for the design of wound dressings because, in practice, the frequency with which a wound dressing needs to be changed depends not on the total amount of fluid that can be absorbed by the whole dressing but on how much fluid (i.e. wound exudate) can be absorbed before some of the fluid penetrates through the dressing. Once the wound exudate has penetrated through any part of the dressing the dressing must normally be changed to avoid soiling clothes, bedding, etc.

Based on the measure of absorbency specified above, the composite fabric of Example 1 was found to be as effective in containing fluid as three 4-ply conventional nonwoven dressings, each ply being 35g/m² (a total weight of conventional dressings of 420g/m²/). In each case, 2.25ml of water was absorbed before leakage through the dressing was observed. The reason for the large improvement in effective absorbent capacity for the composite fabric of Example 1 is that the absorbed liquid is trapped between the hydrophobic layers and spreads (wicks) along and through the core absorbent layers before it penetrates through the lower hydrophobic layer. In this way most or all of the absorptive capacity of the absorbent layers is actually used to absorb liquid, in contrast to the conventional wound dressings in which only a small part of the absorptive capacity is actually used.

The above-described embodiment: are intended by way of example only. Many other embodiments of the composite fabric and the method of manufacture according to the present invention will be apparent to persons skilled in the art.

## Claims

1. An absorbent composite fabric (1,6) formed from a water-permeable hydrophobic layer (2,8) of fibrous material and a water-absorbent fibrous second layer (3,7), the first and second layers being bonded together by entanglement of fibres of the first layer (2,8) in fibres of the second layer (3,7), and characterized in that the first layer (2,8) has a weight of from 15 to 40 g/m².

2. An absorbent composite fabric (1,6) according to claim 1 wherein the entanglement is hydraulic entanglement.

3. An absorbent composite fabric (1,6) according to claim 1 or 2 wherein the first layer (2,8) of fibrous material comprises polypropylene or polyester fibres.

4. An absorbent composite fabric (1,6) according to claim 1, 2 or 3 wherein the fibrous second layer (3,7) comprises water-absorbent staple fibres.

5. An absorbent composite fabric (1,6) according to claim 4 wherein the water-absorbent staple fibres are viscose fibres, modified viscose fibres, trilobal viscose fibres, cotton fibres, solvent-spun cellulose fibres or mixtures of these.

6. An absorbent composite fabric (1,6) according to claim 4 or 5 wherein the fibrous second layer (3,7) further comprises non-water-absorbent staple fibres.

7. An absorbent composite fabric (1,6) according to claim 6 wherein the non-water-absorbent staple fibres are hollow section polyester fibres.

8. An absorbent composite fabric (1,6) according to any preceding claim wherein the weight of the fibrous second layer (3,7) is between 20 and 120g/m².

9. An absorbent composite fabric (1,6) according to any preceding claim wherein the said first layer (2,8) of said composite fabric is hydrophobic at least in part by virtue of a hydrophobic finish applied to the fibres of the first layer (2,8) of fibrous material

10. An absorbent composite fabric (1,6) according to any preceding claim wherein the fibrous second layer comprises antiseptics and/or humectants.

11. An absorbent composite fabric (1,6) according to claim 10 wherein the antiseptic is silver chloride or silver sulphadiazine.

12. An absorbent composite fabric (1,6) according to claim 10 wherein the humectant is glycerol.

13. An absorbent composite fabric (6) according to any preceding claim wherein a further layer (9) or layers of hydrophobic fibrous material is or are bonded to the side of the water-absorbent fibrous second layer (8) opposite the said first layer (7) of fibrous material (6).

14. An absorbent composite fabric (6) according to claim 13 which is formed from two water-permeable first layers (8,9) of fibrous material each having a weight of from 15 to 40 g/m² and a water-absorbent fibrous second layer (7), the first layers (8,9) of said composite fabric being hydrophobic, and the two first layers being bonded one on either side of the second layer (7) by entanglement of fibres of the first layers (8,9) in fibres of the second layer (7).

15. An absorbent composite fabric (6) according to claim 14 wherein the water-absorbent fibrous second layer (7) is a composite layer.

16. A wound dressing or burn dressing comprising an absorbent composite fabric according to any preceding claim.

17. A blanket, sheet, sweat-absorbent lining, medical drape, absorbent feminine hygiene product or incontinence pad comprising an absorbent composite fabric according to any preceding claim.

18. A method of manufacturing absorbent non-woven fabrics, comprising the steps of:
forming a layered web comprising a water-permeable first layer (10) of a first fibrous material and a second layer (11) containing water absorbent fibrous material;
entangling the fibres of the first and second layers of the layered web (12) at the interface between the first and second layers by supporting the layered web (12) on a water permeable mesh (13) with the said second layer closest to the mesh and moving the layered web (12) and mesh relative to a plurality of water jets (14,15,16) while the water jets are directed at the surface of the said first layer (10) of the layered web; and
drying the resulting composite fabric, characterized in that the first layer (1) has a weight of from 15 to 40 g/m².

19. A method according to claim 18 wherein the first and second layers comprise carded webs.

20. A method according to claim 18 or 19 wherein the layered web (12) is transported on the water-permeable mesh (13) substantially flat and in a horizontal direction under a plurality of entangling water jets.

21. A method according to claim 18 or 19 wherein the layered web (12) is supported on a water permeable mesh mounted on an open surface cylinder and the cylinder is rotated relative to water jets located around the circumference of the cylinder.

22. A method according to any of claim 18 to 21 wherein after the drying step a hydrophobic finish is applied to fibres at a surface of the composite fabric.

23. A method according to any of claims 18 to 22 wherein after the entangling step the composite fabric (1,6) undergoes a further entangling step with a further layer of layers of fibrous material.

24. A method according to claim 23 wherein the further layers of fibrous material comprise a second layered web.

## Patentansprüche

1. Absorbierender Textilverbundstoff (1, 6), gebildet aus einer wasserdurchlässigen, hydrophoben Schicht (2, 8) aus faserigem Material und einer wasserabsorbierenden, faserigen zweiten Schicht (3, 7), wobei die erste und die zweite Schicht miteinander durch Verschlingung der Fasern der ersten Schicht (2, 8) mit den Fasern der zweiten Schicht (3, 7) verbunden sind, dadurch gekennzeichnet, daß die erste Schicht (2, 8) ein Gewicht von 15 bis 40 g/m² aufweist.

2. Absorbierender Textilverbundstoff (1, 6) nach Anspruch 1, wobei es sich bei der Verschlingung um eine hydraulische Verschlingung handelt.

3. Absorbierender Textilverbundstoff (1, 6) nach Anspruch 1 oder 2, wobei die erste Schicht (2, 8) aus faserigem Material Polypropylen- oder Polyester-Fasern umfaßt.

4. Absorbierender Textilverbundstoff (1, 6) nach Anspruch 1, 2 oder 3, wobei die faserige zweite Schicht (3, 7) wasserabsorbierende Stapelfasern umfaßt.

5. Absorbierender Textilverbundstoff (1, 6) nach Anspruch 4, wobei es sich bei den wasserabsorbierenden Stapelfasern um Viskosefasern, modifizierte Viskosefasern, trilobale Viskosefasern, Baumwollfasern, durch Lösungsspinnen hergestellte Cellulosefasern oder Gemische dieser Fasern handelt.

6. Absorbierender Textilverbundstoff (1, 6) nach Anspruch 4 oder 5, wobei die faserige zweite Schicht (3, 7) ferner nicht-wasserabsorbierende Stapelfasern umfaßt.

7. Absorbierender Textilverbundstoff (1, 6) nach Anspruch 6, wobei es sich bei den nicht-wasserabsorbierenden Stapelfasern um Polyesterfasern mit hohlem Querschnitt handelt.

8. Absorbierender Textilverbundstoff (1, 6) nach einem der vorstehenden Ansprüche, wobei das Gewicht der faserigen zweiten Schicht (3, 7) 20 bis 120 g/m² beträgt.

9. Absorbierender Textilverbundstoff (1, 6) nach einem der vorstehenden Ansprüche, wobei die erste Schicht (2, 8) des Textilverbundstoffs aufgrund einer auf die Fasern der ersten Schicht (2, 8) aus faserigem Material aufgebrachten hydrophoben Appretur zumindest teilweise hydrophob ist.

10. Absorbierender Textilverbundstoff (1, 6) nach einem der vorstehenden Ansprüche, wobei die faserige zweite Schicht antiseptische und/oder feuchthaltende Mittel umfaßt.

11. Absorbierender Textilverbundstoff (1, 6) nach Anspruch 10, wobei es sich beim antiseptischen Mittel um Silberchlorid oder Silbersulfadiazin handelt.

12. Absorbierender Textilverbundstoff (1, 6) nach Anspruch 10, wobei es sich beim Feuchthaltemittel um Glycerin handelt.

13. Absorbierender Textilverbundstoff (6) nach einem der vorstehenden Ansprüche, wobei eine weitere Schicht (9) oder Schichten aus einem hydrophoben, faserigen Material an die Seite der wasserabsorbierenden, faserigen zweiten Schicht (8) gegenüber der ersten Schicht (7) des faserigen Materials (6) gebunden sind.

14. Absorbierender Textilverbundstoff (6) nach Anspruch 13, der aus zwei wasserdurchlässigen ersten Schichten (8, 9) aus faserigem Material, die jeweils ein Gewicht von 15-40 g/m² aufweisen, und einer wasserabsorbierenden, faserigen zweiten Schicht (7) gebildet ist, wobei die ersten Schichten (8, 9) des Textilverbundstoffs hydrophob sind und die beiden ersten Schichten an beide Seiten der zweiten Schicht (7) durch Verschlingung der Fasern der ersten Schichten (8, 9) mit den Fasern der zweiten Schicht (7) gebunden sind.

15. Absorbierender Textilverbundstoff (6) nach Anspruch 14, wobei es sich bei der wasserabsorbierenden, faserigen zweiten Schicht (7) um eine Verbundschicht handelt.

16. Wundverband oder Brandbindenverband, umfassend einen absorbierenden Textilverbundstoff nach einem der vorstehenden Ansprüche.

17. Bettdecke, Bettuch, schweißabsorbierende Einlage, medizinisches Tuch, absorbierendes Produkt für die Hygiene der Frau oder Inkontinenzkissen, umfassend einen absorbierenden Textilverbundstoff nach einem der vorstehenden Ansprüche.

18. Verfahren zur Herstellung von absorbierenden Vliesstoffen, umfassend folgende Stufen:
das Bilden einer schichtförmigen Bahn, die eine wasserdurchlässige erste Schicht (10) aus einem ersten faserigen Material und eine zweite Schicht (11) mit einem Gehalt an einem wasserabsorbierenden, faserigen Material umfaßt;
das Verschlingen der Fasern der ersten und der zweiten Schicht der faserigen Bahn (12) an der Grenzfläche zwischen der ersten und der zweiten Schicht, indem man die schichtförmige Bahn (12) auf ein wasserdurchlässiges Sieb (13) so legt, daß sich die zweite Schicht dem Sieb am nächsten befindet, und das Bewegen der schichtförmigen Bahn (12) und des Siebs relativ zu einer Mehrzahl von Wasserstrahldüsen (14, 15, 16), wobei die Wasserstrahldüsen auf die Oberfläche der ersten Schicht (10) der schichtförmigen Bahn gerichtet sind; und
das Trocknen des erhaltenen Textilverbundstoffs, dadurch gekennzeichnet, daß die erste Schicht (1) ein Gewicht von 15-40 g/m² aufweist.

19. Verfahren nach Anspruch 18, wobei die erste und zweite Schicht kardierte Bahnen umfaßt.

20. Verfahren nach Anspruch 18 oder 19, wobei die schichtförmige Bahn (12) auf dem wasserdurchlässigen Sieb (13) in einem im wesentlichen flachem Zustand und in einer horizontalen Richtung unter einer Mehrzahl von eine Verschlingung bewirkenden Wasserstrahldüsen transportiert wird.

21. Verfahren nach Anspruch 18 oder 19, wobei die schichtförmige Bahn (12) auf einem wasserdurchlässigen Sieb aufliegt, das auf einem Zylinder mit offener Oberfläche befestigt ist und der Zylinder relativ zu Wasserstrahldüsen, die um den Umfang des Zylinders herum angeordnet sind, gedreht wird.

22. Verfahren nach einem der Ansprüche 18 bis 21, wobei nach der Trocknungsstufe eine hydrophobe Appretur auf die Fasern auf einer Oberfläche des Textilverbundstoffs aufgebracht wird.

23. Verfahren nach einem der Ansprüche 18 bis 22, wobei nach der Verschlingungsstufe der Textilverbundstoff (1, 6) einer weiteren Verschlingungsstufe mit einer oder mehreren weiteren Schichten aus faserigem Material unterzogen wird.

24. Verfahren nach Anspruch 23, wobei die weiteren Schichten aus faserigem Material eine zweite schichtförmige Bahn umfassen.

## Revendications

1. Un tissu composite absorbant (1, 6) formé à partir d'une couche hydrophobe perméable à l'eau (2, 8) composée de matière fibreuse et une seconde couche fibreuse absorbant l'eau (3, 7), les première et seconde couches étant liées entre elles par embrouillement de fibres de la première couche (2, 8) dans des fibres de la seconde couche (3, 7), et caractérisé en ce que la première couche (2, 8) présente une masse comprise entre 15 et 40 g/m².

2. Un tissu composite absorbant (1, 6) selon la Revendication 1, dans lequel l'embrouillement est un embrouillement hydraulique.

3. Un tissu composite absorbant (1, 6) selon la Revendication 1 ou 2, dans lequel la première couche (2, 8) composée de matière fibreuse comprend des fibres de polypropylène ou de polyester.

4. Un tissu composite absorbant (1, 6) selon la Revendication 1, 2 ou 3, dans lequel la seconde couche fibreuse (3, 7) comprend des fibres textiles découpées absorbant l'eau.

5. Un tissu composite absorbant (1, 6) selon la Revendication 4, dans lequel les fibres textiles découpées absorbant l'eau sont des fibres de viscose, des fibres de viscose modifiée, des fibres de viscose trilobée, des fibres de coton, des fibres de cellulose filée au solvant, ou des mélanges de celles-ci.

6. Un tissu composite absorbant (1, 6) selon la Revendication 4 ou 5, dans lequel la seconde couche fibreuse (3, 7) comprend en outre des fibres textiles découpées n'absorbant pas l'eau.

7. Un tissu composite absorbant (1, 6) selon la Revendication 6, dans lequel les fibres textiles découpées n'absorbant pas l'eau sont des fibres de polyester à section creuse.

8. Un tissu composite absorbant (1, 6) selon l'une quelconque des Revendications précédentes, dans lequel la masse de la seconde couche fibreuse (3, 7) est comprise entre 20 et 120 g/m².

9. Un tissu composite absorbant (1, 6) selon l'une quelconque des Revendications précédentes, dans lequel ladite première couche (2, 8) dudit tissu composite est hydrophobe au moins partiellement du fait d'un apprêt hydrophobe appliqué sur les fibres de la première couche (2, 8) de matière fibreuse.

10. Un tissu composite absorbant (1, 6) selon l'une quelconque des Revendications précédentes, dans lequel la seconde couche fibreuse comprend des agents antiseptiques et/ou humectants.

11. Un tissu composite absorbant (1, 6) selon la Revendication 10, dans lequel l'agent antiseptique est du chlorure d'argent ou de la sulfadiazine d'argent.

12. Un tissu composite absorbant (1, 6) selon la Revendication 10, dans lequel l'agent humectant est du glycérol.

13. Un tissu composite absorbant (1, 6) selon l'une quelconque des Revendications précédentes, dans lequel une autre couche (9) ou d'autres couches de matière fibreuse hydrophobe est ou sont liée(s) à la face de la seconde couche fibreuse absorbant l'eau (8) opposée à ladite première couche (7) de matière fibreuse (6).

14. Un tissu composite absorbant (6) selon la Revendication 13, qui est formé à partir de deux premières couches perméables à l'eau (8, 9) composées de matière fibreuse, chacune ayant une masse comprise entre 15 et 40 g/m² et une seconde couche absorbant l'eau (7), les premières couches (8, 9) dudit tissu composite étant hydrophobes, et les deux premières couches étant liées l'une sur l'un ou l'autre côté de la seconde couche (7) par embrouillement des fibres des premières couches (8, 9) dans des fibres de la seconde couche (7).

15. Un tissu composite absorbant (6) selon la Revendication 14, dans lequel la seconde couche fibreuse absorbant l'eau (7) est une couche composite.

16. Un pansement pour plaies ou pour brûlures comprenant un tissu composite absorbant selon l'une quelconque des Revendications précédentes.

17. Une couverture, un drap, une garniture absorbant la transpiration, des étoffes à usage médical, un produit d'hygiène féminine ou une couche pour incontinence comprenant un tissu composite absorbant selon l'une quelconque des Revendications précédentes.

18. Un procédé de fabrication de tissus non tissés absorbants, comprenant les étapes consistant :
à former une bande en couches comprenant une première couche perméable à l'eau (10) composée d'une première matière fibreuse et une seconde couche (11) contenant une matière fibreuse absorbant l'eau ;
à embrouiller les fibres des première et seconde couches de la bande en couches (12) au niveau de l'interface entre les première et seconde couches en supportant la bande en couches (12) sur un maillage perméable à l'eau (13), ladite seconde couche étant au plus près du maillage et à déplacer la bande en couches (12) et le maillage par rapport à une pluralité de jets d'eau (14, 15, 16) alors que les jets d'eau sont dirigés sur la surface de ladite première couche (10) de la bande en couches ; et
à sécher le tissu composite ainsi obtenu, caractérisé en ce que la première couche (1) présente une masse comprise entre 15 et 40 g/m².

19. Un procédé selon la Revendication 18, dans lequel les première et seconde couches comprennent des bandes cardées.

20. Un procédé selon la Revendication 18 ou 19, dans lequel la bande en couches (12) est transportée sur un maillage perméable à l'eau (13) substantiellement plat et selon une direction horizontale sous une pluralité de jets d'eau d'embrouillement.

21. Un procédé selon la Revendication 18 ou 19, dans lequel la bande en couches (12) est supportée sur un maillage perméable à l'eau monté sur un cylindre à surface ouverte et dans lequel on fait tourner le cylindre par rapport aux jets d'eau situés en périphérie du cylindre.

22. Un procédé selon l'une des Revendications 18 à 21, dans lequel après l'étape de séchage, un apprêt hydrophobe est appliqué sur les fibres sur une surface du tissu composite.

23. Un procédé selon l'une des Revendications 18 à 22, dans lequel après l'étape d'embrouillement, le tissu composite (1, 6) est soumis à une étape d'embrouillement supplémentaire avec une autre couche des couches de matière fibreuse.

24. Un procédé selon la Revendication 23, dans lequel les couches supplémentaires composées de matière fibreuse comprennent une seconde bande en couches.
